# EUROPÄISCHE PATENTANMELDUNG

(11) **EP 4 040 140 A1**
(43) Veröffentlichungstag der Anmeldung: **10.08.2022**
(21) Anmeldenummer: 22155347.2
(22) Anmeldetag: 07.02.2022
(51) Int. Cl.: G01N 21/90, A61M 5/34

(54) **VERFAHREN UND VORRICHTUNG ZUR ÜBERPRÜFUNG EINER KLEBSTOFFVERBINDUNG ZWISCHEN EINER HOHLNADEL ODER KANÜLE UND EINEM HALTETEIL**

(30) Priorität: 08.02.2021 DE 102021102830
(71) Anmelder: ATS Automation Tooling Systems GmbH, 71364 Winnenden (DE)
(72) Erfinder: Haller, Florian, 71364 Winnenden (DE); Lindner, Roland, 71364 Winnenden (DE)
(74) Vertreter: Lichti - Patentanwälte Partnerschaft mbB

(57) **Zusammenfassung**

Es ist ein Verfahren zur Überprüfung einer Klebstoffverbindung zwischen einer Hohlnadel oder Kanüle einer medizinischen Spritze, und einem Halteteil beschrieben, wobei die Hohlnadel oder Kanüle mit Spiel in einem Halteabschnitt des Halteteils eingesetzt und ein Zwischenraum zwischen der Hohlnadel oder Kanüle und dem Halteabschnitt mit einem Klebstoffkörper zumindest teilweise gefüllt ist. Der Klebstoffkörper wird mittels einer optischen Erfassungsvorrichtung in Form einer Kamera erfasst und die Erfassungsvorrichtung gibt Bilddaten bezüglich des Klebstoffkörpers an eine Auswerte-Einheit ab, in der die Daten ausgewertet werden. Aus den Bilddaten des Klebstoffkörpers wird das IST-Volumen und/oder die IST-Konfiguration des Klebstoffkörpers ermittelt und mit einem SOLL-Volumen und/oder einer SOLL-Konfiguration des Klebstoffkörpers verglichen.

## Beschreibung

Die Erfindung betrifft ein Verfahren zur Überprüfung einer Klebstoffverbindung zwischen einer Hohlnadel oder Kanüle und einem Halteteil, wobei die Hohlnadel oder Kanüle mit Spiel in einen Halteabschnitt des Halteteils eingesetzt und ein Zwischenraum zwischen der Hohlnadel oder Kanüle und dem Halteabschnitt mit einem Klebstoffkörper zumindest teilweise gefüllt ist.

Darüber hinaus betrifft die Erfindung eine entsprechende Vorrichtung zur Überprüfung der Klebstoffverbindung zwischen einer Hohlnadel oder Kanüle und dem Halteteil.

Eine vorgefertigte Hohlnadel oder Kanüle für eine medizinische Spritze wird zu ihrer Befestigung in ein rohrförmiges Halteteil eingeklebt, das aus Glas oder Kunststoff besteht. Das Halteteil kann einstückiger Bestandteil des Spritzenkörpers sein, es ist jedoch auch möglich, dass das Halteteil seinerseits auf den Spritzenkörper aufgesetzt und dort beispielsweise verklemmt oder verrastet wird.

Bei einer Hohlnadel oder einer Kanüle handelt es sich üblicherweise um ein eigenstabiles, geradliniges Rohr aus Edelstahl oder Kunststoff. Das Halteteil besitzt einen rohrförmigen Halteabschnitt, dessen Innendurchmesser größer als der Außendurchmesser der Hohlnadel oder Kanüle ist. Wenn die Hohlnadel oder Kanüle in den rohrförmigen Halteabschnitt des Halteteils eingesetzt wird, verbleibt zwischen der Außenseite der Hohlnadel oder Kanüle und der Innenwandung des Halteabschnitts ein Zwischenraum. In diesem Zwischenraum wird von der Außenseite ein Klebstoff eingefüllt, der sich einerseits mit der Außenoberfläche der Hohlnadel oder Kanüle und andererseits mit der Innenwandung des Halteabschnitts über Adhäsion verbindet.

Bei Gebrauch einer medizinischen Spritze muss sichergestellt sein, dass die Hohlnadel oder Kanüle sich von dem Halteteil nicht lösen kann. Es ist deshalb bekannt, die Klebstoffverbindung zwischen der Hohlnadel oder Kanüle und dem Halteteil zu überprüfen, indem das Halteteil fixiert und auf die Hohlnadel oder Kanüle eine Zugkraft ausgeübt wird, die auf die Klebstoffverbindung einwirkt. Dabei besteht die Gefahr, dass die Zugkraft die Klebstoffverbindung beschädigt, so dass sich die Hohlnadel oder Kanüle bei Gebrauch der Spritze von dem Halteteil lösen kann.

Der Erfindung liegt die Aufgabe zugrunde, ein Verfahren zur Überprüfung einer Klebstoffverbindung zwischen einer Hohlnadel oder Kanüle und einem Halteteil zu schaffen, mit dem zuverlässig und zerstörungsfrei eine Bewertung der Klebstoffverbindung erfolgen kann.

Darüber hinaus soll eine entsprechende Vorrichtung zur Überprüfung der Klebstoffverbindung geschaffen werden.

Diese Aufgabe wird erfindungsgemäß hinsichtlich des Verfahrens mit einem Verfahren mit den Merkmalen des Anspruchs 1 gelöst. Dabei wird der Klebstoffkörper mittels einer optischen Erfassungsvorrichtung erfasst. Die von der Erfassungsvorrichtung erfassten Daten bezüglich des Klebstoffkörpers werden an eine Auswerte-Einheit abgegeben, in der die Daten ausgewertet werden. Bei der Erfassungsverrichtung handelt es sich um eine Kamera, mit der Bilddaten des Klebstoffkörpers erzeugt werden.

Als Halteteil findet vorzugsweise ein Glaskörper oder eine Glasspritze Verwendung.

Aus den Bilddaten des Klebstoffkörpers wird das IST-Volumen und/oder die IST-Konfiguration des Klebstoffkörpers ermittelt oder errechnet. Aus dem IST-Volumen und/oder der IST-Konfiguration des Klebstoffkörpers lassen sich Rückschlüsse auf die Kontaktfläche zwischen dem Klebstoffkörper und der Hohlnadel oder Kanüle und auch auf die Größe der Kontaktfläche zwischen dem Klebstoffkörper und der Innenwandung des Halteabschnitts des Halteteils ziehen, woraus sich wiederrum die zu übertragende Kraft und somit die Stabilität der Klebstoffverbindung ableiten lassen. Ferner kann die axiale Eindringtiefe des Klebstoffs in den Zwischenraum zwischen der Hohlnadel oder Kanüle und der Wandung des Halteteils ermittelt werden. Desweiteren lassen sich eventuelle Lufteinschlüsse im Klebstoffkörper feststellen.

Dabei werden das IST-Volumen und/oder die IST-Konfiguration des Klebstoffkörpers in der Auswerte-Einheit mit einem SOLL-Volumen und/oder einer SOLL-Konfiguration des Klebstoffkörpers verglichen. Wenn eine Abweichung zwischen den IST-Volumen und dem SOLL-Volumen und/oder eine Abweichung zwischen der IST-Konfiguration und der SOLL-Konfiguration außerhalb vorgegebener Grenzen liegt, wird die Klebstoffverbindung als ungenügend eingestuft und aus dem weiteren Produktionsprozess aussortiert. Zusätzlich oder alternativ dazu kann die Klebstoffmenge für die zukünftigen Klebstoffkörper auf der Basis der erfassten Bilddaten angepasst, d.h. erhöht und/oder vermindert werden.

Die Zuverlässigkeit des Verfahrens hängt wesentlich von der Qualität der Daten ab, die von der optischen Erfassungsvorrichtung von dem Klebstoffkörper erfasst werden. Wenn das Halteteil oder zumindest der Halteabschnitt aus Glas, einem zumindest teilweise lichtdurchlässigen Kunststoff oder einem anderen lichtdurchlässigen Material besteht, kann es ausreichen, von der Hohlnadel oder Kanüle und dem Halteteil ein Foto des Klebstoffkörpers von der Außenseite des Halteteils aufzunehmen. Dabei können die aufzunehmenden Bauteile oder Bauteil-Abschnitte durch zumindest eine Lichtquelle beleuchtet sein.

In Weiterbildung der Erfindung ist vorgesehen, dass der Klebstoffkörper bzw. der den Klebstoffkörper bildende Klebstoff zumindest teilweise aus einem fluoreszierenden Material besteht und/oder fluoreszierende Partikel enthält. Wenn das fluoreszierende Material oder die fluoreszierende Partikel vor der Erfassung durch die Erfassungsvorrichtung beispielsweise durch Beleuchtung angeregt werden, leuchten Sie für einen kurzen Zeitraum und heben sich auf diese Weise deutlich von benachbarten Bauteilen ab, wodurch sie sich mit hoher Genauigkeit fototechnisch erfassen lassen. Vorzugsweise ist vorgesehen, dass der Klebstoffkörper vor und/oder während und/oder nach der Erfassung mittels der optischen Erfassungsvorrichtung von einer Beleuchtungsvorrichtung mit zumindest einer Lichtquelle beleuchtet wird. Vorzugsweise wird der Klebstoffkörper mit Licht mit einer Wellenlänge ≤ 500nm und insbesondere mit Licht mit einer Wellenlänge ≤ 405nm beleuchtet. Es hat sich als vorteilhaft erwiesen, den Klebstoffkörper mit Licht mit einer Wellenlänge im Bereich von 360nm bis 370nm zu beleuchten.

Als Lichtquelle kann zumindest eine Leuchtdiode (LED) verwendet werden.

Als Kamera kann eine handelsübliche, vorzugsweise hochauflösende Kamera verwendet werden, mit der auch sehr kurze Belichtungsseiten im Bereich von 20ps bis ls möglich sein sollten.

Wenn das Halteteil aus Glas oder einem anderen stark reflektierenden Werkstoff besteht, besteht die Gefahr, dass sich auf der Oberfläche des Halteteils Lichtreflektionen bilden, die von der optischen Erfassungsvorrichtung auch erfasst werden und die Auswertung stören können. Vorzugsweise ist deshalb vorgesehen, dass die Bilddaten, die Bereiche des Halteteils betreffen, in denen starke Licht-Reflektionen auftreten können, bei der Auswertung außer Acht gelassen werden. Dies kann erreicht werden, indem die Auswerte-Einheit die erfassten Bilddaten in verschiedene Bildbereiche segmentiert und insbesondere diejenigen Bereiche für die Auswertung ausblendet, in denen entweder Licht-Reflektionen erkannt werden und/oder von denen bekannt ist, dass dort häufig Licht-Reflektionen auftauchen.

Bei der Bildverarbeitung kann das Bild zusätzlich noch mit einem "Binning" vorbereitet werden, um die Helligkeitswerte der Kameraauflösung zu erhöhen. Bei einem "Binning" werden die Helligkeitswerte von einer bestimmten Anzahl benachbarter Pixel aufaddiert und jene Pixel zu einem Pixel zusammengefasst. Dadurch wird eine schwache Fluoreszenz des Material des Klebstoffkörpers besser sichtbar.

Um stark reflektierende Bereiche des Halteteils und/oder der Hohlnadel oder Kanüle zu ermitteln, kann vorgesehen sein, in einem vorgelagerten Verfahrensschritt ein Halteteil und eine Hohlnadel oder Kanüle ohne einen Klebstoffkörper zu erfassen und die entsprechenden Fotos auszuwerten. Dadurch lassen sich Bildbereiche feststellen, in denen das Licht reflektiert wird und die nicht auf die Leuchtkraft des Klebstoffkörpers zurückgehen, da hierbei noch kein Klebstoffkörper vorhanden ist. Die festgestellten Reflektionsbereiche werden bei der Auswertung der Bilddaten des Halteteils mit Klebstoffkörper und mit Hohlnadel oder Kanüle vorzugsweise außer Acht gelassen oder ausgeblendet. Auf diese Weise lassen sich diejenigen Bildbereiche ermitteln, die für die Auswertung herangezogen werden.

Wenn das Halteteil rohrförmig ausgebildet ist und die Hohlnadel oder Kanüle von einem axialen Ende in den Innenraum des rohrförmigen Halteteils eingesteckt wird, kann die Lichtquelle auf der axial entgegengesetzten Seite des Halteteils angeordnet sein, so dass vorgesehen sein kann, dass der Klebstoffkörper durch den Innenraum des Halteteils hindurchbeleuchtet wird. Aufgrund der Einbringung des Lichtes durch den Innenraum des Halteteils lassen sich Reflektionen reduzieren. Je weniger Reflektionen vorhanden sind, desto besser kann die relativ schwache Fluoreszenz des Klebstoffkörpers erfasst werden. Aus diesem Grund sollten vorzugsweise auch weitere externe, störende Lichtquellen abgeschirmt werden.

Alternativ oder zusätzlich dazu kann vorgesehen sein, dass der Klebstoffkörper durch das Material des Halteteils hindurch beleuchtet wird.

In einer möglichen Ausgestaltung der Erfindung kann vorgesehen sein, dass der den Klebstoffkörper bildende Klebstoff ein unter Licht aushärtender Klebstoff ist. Auf diese Weise ist es möglich, das Aushärten des Klebstoffs und die Beleuchtung des Klebstoffkörpers für die Erfassung durch die optische Erfassungsvorrichtung zu verbinden. Insbesondere kann vorgesehen sein, dass die Aushärtung des Klebstoffs zumindest teilweise und vorzugsweise vollständig synchron mit der Beleuchtung des Klebstoffkörpers durch die Lichtquelle erfolgt.

In einer möglichen Ausgestaltung der Erfindung kann vorgesehen sein, dass der Klebstoff zunächst vorgehärtet wird. Dies kann durch Aufbringung von Licht mittels einer stromauf liegenden 1. Lichtquelle erfolgen, wodurch der Klebstoff zusätzlich mit Lichtenergie aufgeladen wird. Nach der Vorhärtung des Klebstoffs erfolgt die Erfassung des Klebstoffkörpers mittels der optischen Erfassungsvorrichtung, wobei die 1. Lichtquelle und/oder eine 2. Lichtquelle während der Erfassung vorzugsweise angeschaltet bleiben oder auf eine verringerte Leistung reduziert werden. Bei Reduzierung der Leistung der 1. und/oder 2. Lichtquelle kann erreicht werden, dass das von der 1. und/oder 2. Lichtquelle ausgehende Licht und eventuell daraus resultierende Licht-Reflektionen die Erfassung des Klebstoffkörpers durch die optische Erfassungsvorrichtung nicht beinträchtigen.

Das Vorhärten und die optische Erfassung des Klebstoffkörpers können in einer gemeinsamen Arbeitsstation erfolgen. Es ist jedoch auch möglich, das vorher Vorhärten und die optische Erfassung des Klebstoffkörpers in separaten Arbeitsstationen nacheinander oder gleichzeitig oder mit seitlicher Überlappung durchzuführen. Für das Vorhärten kann der Klebstoffkörper für eine Dauer von ls bis 6s beleuchtet werden.

Sobald die Erfassung des Klebstoffkörpers durch die optische Erfassungsvorrichtung abgeschlossen ist, erfolgt die Aushärtung des Klebstoffs, indem beispielsweise die 1. und/oder 2. Lichtquelle und/oder eine weitere 3. Lichtquelle aktiviert und der Klebstoffkörper dem Licht ausgesetzt und dadurch ausgehärtet wird.

In bevorzugter Ausgestaltung der Erfindung ist vorgesehen, dass zumindest die optische Erfassung des Klebstoffkörpers in einer Dunkelkammer durchgeführt wird. Unter einer Dunkelkammer ist ein von Wänden umgebener Raum zu verstehen, der gegenüber dem Einfall äußeren Lichtes abgeschirmt ist. Die Dunkelkammer kann eine vorzugsweise verschließbare Eingangsöffnung und/oder eine vorzugsweise verschließbare Ausgangsöffnung aufweisen. Ein Prüfkörper, der das Halteteil, die Hohlnadel oder Kanüle und den Klebstoffkörper umfasst, wird in die Dunkelkammer eingebracht und ist in dieser gegenüber äußerem Licht abgeschirmt. Der Prüfkörper wird in gewünschter Weise positioniert und dann wird die entsprechende Lichtquelle aktiviert. Anschließend erfolgt die Bildaufnahme mit einer Belichtungszeit von vorzugsweise 0,01s mit 0,02s. Die Lichtquelle wird vorzugsweise vor der Aufnahme des Bildes angestellt und erst nach erfolgter Bildaufnahme wieder abgestellt, wobei die Aktivierung der Lichtquelle für eine Dauer von beispielsweise 0,1s bis 0,8s und vorzugsweise von 0,5s erfolgen kann.

Das Vorhärten des Klebstoffkörpers kann ebenfalls in der Dunkelkammer erfolgen, es ist jedoch auch möglich, das Vorhärten außerhalb der Dunkelkammer durchzuführen, d.h. bevor der Prüfkörper in die Dunkelkammer einfährt.

Auch das nachgeschaltete Aushärten des Klebstoffkörpers kann in der Dunkelkammer folgen, d.h. bevor der Prüfkörper vorzugsweise durch eine verschließbare Ausgangsöffnung aus der Dunkelkammer herausfährt. Alternativ ist es jedoch auch möglich, das Aushärten des Klebstoffkörpers erst nach Verlassen der Dunkelkammer durchzuführen.

Eine Vorrichtung zur Überprüfung einer Klebstoffverbindung zwischen einer Hohlnadel oder Kanüle und einem Halteteil zeichnet sich durch eine optische Erfassungsvorrichtung aus, mittels der der Klebstoffkörper erfassbar ist. Darüber hinaus ist eine Auswerte-Einheit vorgesehen, der Daten bezüglich des Klebstoffkörpers von der Erfassungsvorrichtung zuführbar sind. Die Erfassungsvorrichtung weist eine Kamera auf, mit der Bilddaten des Klebstoffkörpers generierbar sind.

Weitere Merkmale der Vorrichtung ergeben sich aus der vorstehenden Erläuterung des Verfahrens, wobei die im Zusammenhang mit dem Verfahren dargestellten vorrichtungstechnischen Merkmale einzeln oder in beliebiger Kombination miteinander vorgesehen sein können.

Weitere Einzelheiten und Merkmale der Erfindung sind aus der folgenden Beschreibung eines Ausführungsbeispiels unter Bezugnahme auf die Zeichnungen ersichtlich.

Es zeigen:
- Fig. 1: eine schematische perspektivische Ansicht einer Vorrichtung zur Überprüfung einer Klebstoffverbindung,
- Fig. 2: einen Längsschnitt durch einen Prüfkörper und
- Fig. 3: eine Fig. 2 entsprechende Darstellung mit einzelnen Bildbereichen.

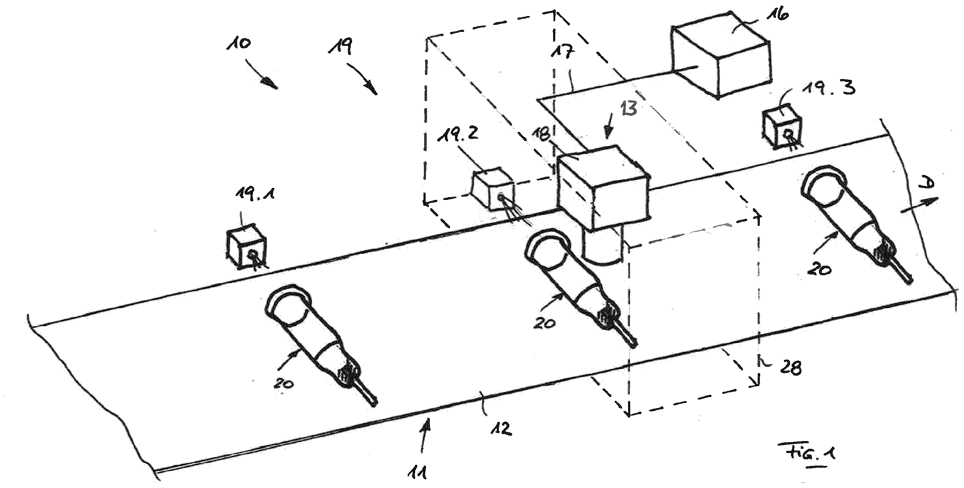

Fig. 1 zeigt eine Vorrichtung 10 zur Überprüfung einer Klebstoffverbindung an Prüfkörpern 20. Die Prüfkörper 20 liegen auf einer Fördervorrichtung 11 in Form eines Förderbandes 12, das gemäß Fig. 1 nach rechts bewegt wird, wie es durch den Pfeil D angedeutet ist. Auf dem Förderband 12 sind die Prüfkörper 20 in einer Reihe in gegenseitigem Abstand und in vorbestimmter Ausrichtung und Positionierung angeordnet.

Oberhalb des Förderbandes 12 ist eine optische Erfassungsvorrichtung 13 positioniert. Die optische Erfassungsvorrichtung 13 besitzt eine Kamera 18, die Bilddaten des jeweils unter ihr befindlichen Prüfkörpers 20 aufnehmen kann. Die Kamera 18 ist über eine Leitung 17 mit einer Auswerte-Einheit 16 verbunden.

Seitlich neben dem Förderband 12 ist eine Beleuchtungsvorrichtung 19 mit mehreren Lichtquellen 19.1, 19.2, 19.3 in Form von Leuchtdioden angeordnet, wobei eine 1. Lichtquelle 19.1 stromauf der Kamera 18 und eine 3. Lichtquelle 19.3 stromab der Kamera 18 angeordnet ist. Eine weitere 2. Lichtquelle 19.2 beleuchtet den Prüfkörper 20, wenn dieser unterhalb der Kamera 18 angeordnet ist.

Es ist eine Dunkelkammer 28 vorgesehen, die in Figur 1 nur schematisch angedeutet ist und in der die optische Erfassungsvorrichtung 13 und die Kamera 18 positioniert sind. Die Prüfkörper 20 können in die Dunkelkammer 28 vorzugsweise durch eine verschließbare Eingangsöffnung oder eine Schleuse einfahren und sind in der Dunkelkammer 28 gegenüber der Umgebung abgeschirmt. Die 2. Lichtquelle 19.2, die den Prüfkörper 20 beleuchtet, wenn er unterhalb der Kamera 18 angeordnet ist, ist ebenfalls innerhalb der Dunkelkammer 28 angeordnet. Nach Erfassung durch die optische Erfassungsvorrichtung 13 kann der Prüfkörper 20 vorzugsweise durch eine verschließbare Ausgangsöffnung oder eine Schleuse aus der Dunkelkammer 28 ausfahren.

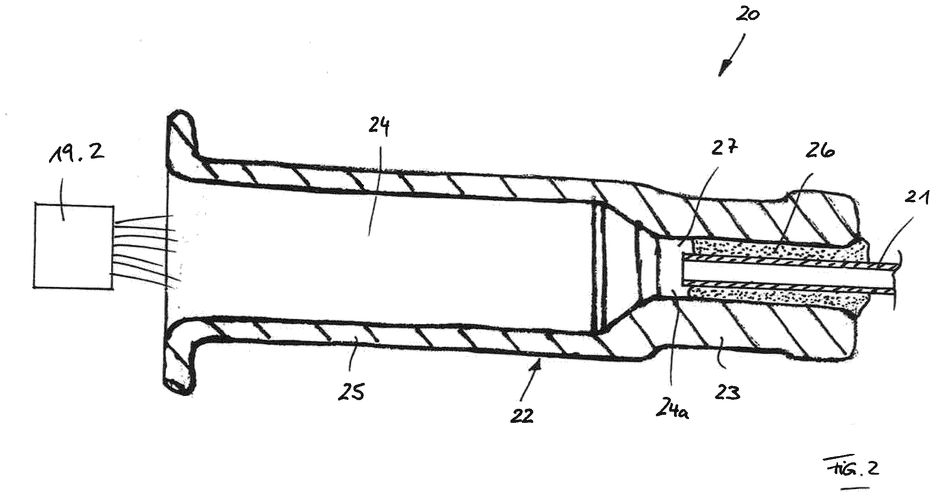

Fig. 2 zeigt einen Längsschnitt durch den Prüfkörper 20. Der Prüfkörper 20 weist ein Halteteil 22 aus Glas oder einem transparenten Kunststoff auf. Das Halteteil 22 ist rohrförmig ausgebildet und besitzt einen Innenraum 24, der sich durch die gesamte axiale Länge des Halteteils 22 erstreckt. In dem gemäß Fig. 2 rechten Endbereich des Halteteils 22 ist an diesem ein Halteabschnitt 23 ausgebildet. Der Halteabschnitt 23 zeichnet sich dadurch aus, dass die Innenabmessungen des Innenraums 24 unter Bildung eines Befestigungsraums 24a verringert sind. In dem gemäß Fig. 2 linkem Endbereich des Halteteils 22 ist an diesem ein Aufsetzabschnitt 25 gebildet ist, mit dem das Halteteil 22 beispielsweise auf einen Spritzenkörper einer medizinischen Spritze aufgesetzt werden kann.

Eine Hohlnadel oder Kanüle 21 ist in axialer Richtung in den Befestigungsraum 24a mit Spiel eingesetzt, so dass zwischen der Außenoberfläche der Hohlnadel oder Kanüle 21 und der Innenwandung des Befestigungsraums 24a ein Zwischenraum 27 gebildeten ist, in den Klebstoff eingefüllt, der einen Klebstoffkörper 26 bildet.

An dem der Hohlnadel oder Kanüle 21 abgewandten axialen Ende, d.h. gemäß Fig. 2 auf der linken Seite des Halteteils 22 ist die 2. Lichtquelle 19.2 angeordnet, deren Licht durch den Innenraum 24 des Halteteils 21 auf den Klebstoffkörper 26 gerichtet ist, auf diesen einwirkt und diesen aushärtet.

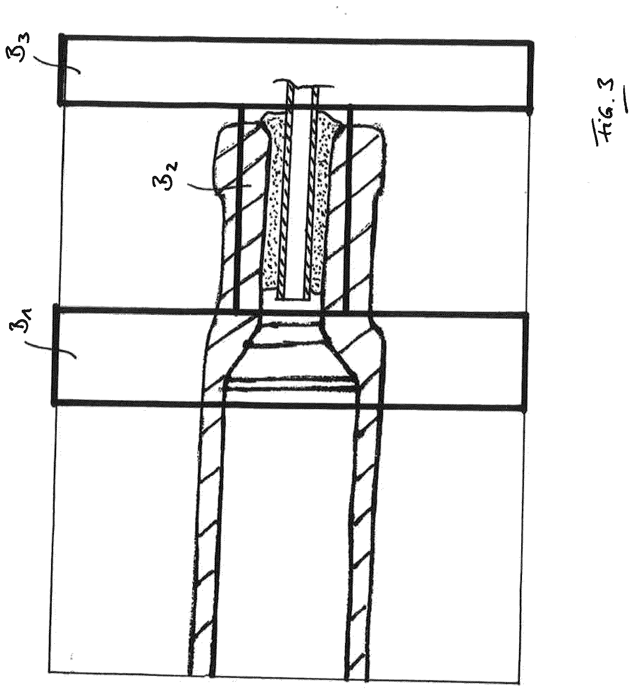

Fig. 3 zeigt beispielhaft ein Bild, das mittels der Kamera 18 von einem Teil des Prüfkörpers 20 aufgenommen wird. Einzelne Bereiche B₁ und B₃ des Bildes werden bei der Auswertung zur Ermittlung der Größe bzw. des Volumens des Klebstoffkörpers 26 sowie der Ermittlung der Lage und Konfiguration des Klebstoffkörpers 26 außer Acht gelassen, da in diesen Bereichen B₁ und B₃ erfahrungsgemäß erhöhte Licht-Reflektionen auftauchen. In die Auswertung des Bildes gehen nur bevorzugte Bereiche B2 ein, die durch die SOLL-Konfiguration des Klebstoffkörpers 26 bestimmt sind.

Zur Überprüfung der Klebstoffverbindung wird der zu überprüfende Prüfkörper 20 auf das Förderband 12 gelegt. Kurz bevor der Prüfkörper 20 eine Position unterhalb der Kamera 18 erreicht, wird er mit Licht aus der stromauf der Kamera 18 angeordneten 1. Lichtquelle 19.1 beaufschlagt. Dies führt dazu, dass der den Klebstoffkörper 26 bildende Klebstoff vorgehärtet wird. Der Prüfkörper 20 fährt dann in die Dunkelkammer 28 ein.

Wenn der zu untersuchende Prüfkörper 20 eine Position unterhalb der Kamera 18 erreicht hat, findet eine Beleuchtung mittels der 2. Lichtquelle 19.2 statt, wodurch der Klebstoffkörper 26 mit Lichtenergie geladen wird, die er dann im folgenden zeitversetzt wieder abgibt, insbesondere wenn der Klebstoff zumindest zum Teil aus einem fluoreszierenden Material besteht oder zumindest fluoreszierende Partikel enthält. Der Klebstoffkörper 26 leuchtet infolge seiner Fluoreszenz und kann auf diese Weise auf einem Foto, das mittels der Kamera 18 gemacht wird, mit hoher Genauigkeit identifiziert und ausgewertet werden.

Nach der Erfassung des Klebstoffkörpers 26 mittels der Kamera 18, wird der Klebstoffkörper 26 aus der Dunkelkammer 28 herausgefahren und anschließend in einer Position stromab der Kamera 18 erneut mit Licht der 3. Lichtquelle 19.3 beaufschlagt, wodurch eine Aushärtung des Klebstoffkörpers 26 erfolgt.

In Abhängigkeit von der Auswertung der mittels der Kamera 18 erfassten Bilddaten des Klebstoffkörpers 26 durch die Auswerte-Einheit 16 wird der Prüfkörper 20 entweder zur weiteren Bearbeitung zugelassen oder aus dem Fertigungsprozess als fehlerhaft aussortiert.

## Patentansprüche

1. Verfahren zur Überprüfung einer Klebstoffverbindung zwischen einer Hohlnadel oder Kanüle (21) und einem Halteteil (22), wobei die Hohlnadel oder Kanüle (21) mit Spiel in einen Halteabschnitt (23) des Halteteils (22) eingesetzt und ein Zwischenraum (27) zwischen der Hohlnadel oder Kanüle (21) und dem Halteabschnitt (23) mit einem Klebstoffkörper (26) zumindest teilweise gefüllt ist, wobei der Klebstoffkörper (26) mittels einer optischen Erfassungsvorrichtung (13) erfasst wird und die Erfassungsvorrichtung (13) Daten bezüglich des Klebstoffkörpers (26) an eine Auswerte-Einheit (16) abgibt, in der die Daten ausgewertet werden, wobei die Erfassungsvorrichtung (13) eine Kamera (18) aufweist, mit der Bilddaten des Klebstoffkörpers (26) erzeugt werden und wobei aus den Bilddaten des Klebstoffkörpers (26) das IST-Volumen und/oder die IST-Konfiguration des Klebstoffkörpers (26) ermittelt oder errechnet wird und mit einem SOLL-Volumen und/oder einer SOLL-Konfiguration des Klebstoffkörpers (26) verglichen wird.

2. Verfahren nach Anspruch 1, **dadurch gekennzeichnet, dass** der Klebstoffkörper (26) zumindest teilweise aus einem fluoreszierenden Material besteht und/oder fluoreszierende Partikel enthält.

3. Verfahren nach Anspruch 1 oder 2, **dadurch gekennzeichnet, dass** der Klebstoffkörper (26) vor und/oder während und/oder nach der Erfassung mittels der optischen Erfassungsvorrichtung (13) von zumindest einer Lichtquelle (19) beleuchtet wird.

4. Verfahren nach Anspruch 3, **dadurch gekennzeichnet, dass** der Klebstoffkörper (26) mit Licht mit einer Wellenlänge ≤ 500nm beleuchtet wird.

5. Verfahren nach Anspruch 4, **dadurch gekennzeichnet, dass** der Klebstoffkörper (26) mit Licht mit einer Wellenlänge ≤ 405nm beleuchtet wird.

6. Verfahren nach Anspruch 5, **dadurch gekennzeichnet, dass** der Klebstoffkörper (26) mit Licht mit einer Wellenlänge im Bereich von 360nm bis 370nm beleuchtet wird.

7. Verfahren nach einem der Ansprüche 3 bis 6, **dadurch gekennzeichnet, dass** als Lichtquelle (29) zumindest eine Leuchtdiode (LED) verwendet wird.

8. Verfahren nach einem der Ansprüche 3 bis 7, **dadurch gekennzeichnet, dass** der Klebstoffkörper (26) durch das Halteteil (22) hindurch beleuchtet wird.

9. Verfahren nach einem der Ansprüche 3 bis 8, **dadurch gekennzeichnet, dass** der den Klebstoffkörper (26) bildende Klebstoff ein unter Licht aushärtender Klebstoff ist und dass die Aushärtung des Klebstoffs zumindest teilweise synchron mit der Beleuchtung des Klebstoffkörpers (26) mittels der Lichtquelle (19) erfolgt.

10. Verfahren nach einem der Ansprüche 3 bis 9, **dadurch gekennzeichnet, dass** der Klebstoff zunächst vorgehärtet wird, anschließend die Erfassung des Klebstoffkörpers (26) mittels der optischen Erfassungsvorrichtung (13) erfolgt und dann der Klebstoff ausgehärtet wird.

11. Verfahren nach einem der Ansprüche 1 bis 10, **dadurch gekennzeichnet, dass** die Erfassung des Klebstoffkörpers (26) mittels der optischen Erfassungsvorrichtung (13) in einer Dunkelkammer (28) erfolgt.

12. Verfahren nach Anspruch 11, **dadurch gekennzeichnet, dass** auch die Vorhärtung des Klebstoffs und/oder die Aushärtung des Klebstoffs in der Dunkelkammer (28) erfolgt.

13. Vorrichtung zur Überprüfung einer Klebstoffverbindung zwischen einer Hohlnadel oder Kanüle (21) und einem Halteteil (22), wobei die Hohlnadel oder Kanüle (21) mit Spiel in einem Halteabschnitt (23) des Halteteils (22) eingesetzt und ein Zwischenraum (27) zwischen der Hohlnadel oder Kanüle (21) und dem Halteabschnitt (23) mit einem Klebstoffkörper (26) zumindest teilweise gefüllt ist, **gekennzeichnet durch** eine optische Erfassungsvorrichtung (13), mittels der der Klebstoffkörper (26) erfassbar ist, und eine Auswerte-Einheit (16), der Daten bezüglich des Klebstoffkörpers (26) von der Erfassungsvorrichtung (13) zuführbar sind, wobei die Erfassungsvorrichtung (13) eine Kamera (18) aufweist, mit der Bilddaten des Klebstoffkörpers (26) generierbar sind.

14. Vorrichtung nach Anspruch 13, **gekennzeichnet durch** zumindest eine Beleuchtungsvorrichtung (19), mittels der der Klebstoffkörper (26) zumindest vor und/oder während der Erfassung mittels der optischen Erfassungsvorrichtung (13) beleuchtbar ist.

15. Vorrichtung nach Anspruch 14, **dadurch gekennzeichnet, dass** der Klebstoffkörper (26) mit Licht mit einer Wellenlänge ≤ 500nm und insbesondere ≤ 405nm beleuchtbar ist.

16. Vorrichtung nach Anspruch 15, **dadurch gekennzeichnet, dass** der Klebstoffkörper (26) mit Licht im Bereich von 360nm bis 370nm beleuchtbar ist.

17. Vorrichtung nach einem der Ansprüche 13 bis 16, **dadurch gekennzeichnet, dass** der Klebstoffkörper (26) durch das Halteteil (22) hindurch beleuchtbar ist.

18. Vorrichtung nach einem der Ansprüche 12 bis 17, **gekennzeichnet durch** eine Dunkelkammer (28), in der die optische Erfassungsvorrichtung (13) angeordnet ist.
